# EUROPÄISCHE PATENTSCHRIFT

(11) **EP 3 772 343 B1**
(45) Veröffentlichungstag und Bekanntmachung des Hinweises auf die Patenterteilung: **25.08.2021**
(21) Anmeldenummer: 20187715.6
(22) Anmeldetag: 24.07.2020
(51) Int. Cl.: A61B 17/29, A61B 34/00, A61B 17/00

(54) **MEDIZINISCHES INSTRUMENT**
MEDICAL INSTRUMENT
INSTRUMENT MÉDICAL

(30) Priorität: 05.08.2019 DE 102019121092
(43) Veröffentlichungstag der Anmeldung: 10.02.2021
(73) Patentinhaber: Karl Storz SE & Co. KG, 78532 Tuttlingen (DE)
(72) Erfinder: Grüner, Sven, 78532 Tuttlingen (DE)
(74) Vertreter: Hofmeister, Frank

(56) Entgegenhaltungen:
- US-A- 5 454 827
- US-A1- 2005 273 084
- US-A1- 2006 020 287
- US-A1- 2015 321 343
- US-A1- 2015 352 728
- US-A1- 2016 066 982

## Beschreibung

Die Erfindung betrifft ein medizinisches Instrument mit einem hohlen Schaft, einer am proximalen Ende des Schaftes angeordneten Betätigungseinheit und einer am distalen Ende des Schaftes angeordneten Instrumentenspitze mit einem Instrument , wobei das Instrument über ein axial verschiebbar im Schaft gelagertes Betätigungselement betätigbar ist, das proximalseitig mit der Betätigungseinheit in Wirkverbindung steht und die Instrumentenspitze über einen Gelenkmechanismus relativ zur Längsachse des Schaftes verschwenkbar ist, wobei der Gelenkmechanismus aus am distalen Ende des Schaftes angeordneten Schwenkgliedern besteht, die über in Längsrichtung des Schaftes verlaufende Lenkdrähte so mit einem proximalseitigen Antrieb verbunden sind, dass eine Bewegung des proximalseitigen Antriebs eine entsprechende relative Bewegung der distalseitigen Schwenkglieder und somit ein Verschwenken der Instrumentenspitze verursacht und wobei der proximalseitige Antrieb eine räumlich verstellbare Scheibe aufweist, an der die Lenkdrähte gelagert sind.

Schwenkglieder mit drei, vier oder mehr außenliegenden Lenkdrähten/Lenkseilen für abwinkelbare medizinische Instrumente sind aus der Praxis für handgeführte und/oder robotische Instrumente bekannt. Für eine feinfühlige Steuerung des distalen Endes eines derartigen medizinischen Instruments haben sich viele dünne Lenkdrähte/Lenkseile gegenüber wenigen dickeren Lenkdrähten/Lenkseilen als vorteilhafter erwiesen, da so unter anderem eine gleichmäßigere Kraftverteilung in alle Abwinklungsrichtungen zu erzielen ist und darüber hinaus erlauben dünnere Lenkdrähte/Lenkseile mehr Platz im Innenraum für elektrische Leitungen und dergleichen.

Ein gattungsgemäßes medizinisches Instrument ist beispielsweise aus der US 5 454 827 bekannt. Bei diesem bekannten medizinischen Instrument sind die distalseitigen Schwenkglieder über vier Lenkdrähte mit einer proximalseitig angeordneten räumlich verstellbaren Scheibe so gekoppelt, dass eine Bewegung der räumlich verstellbaren Scheibe eine entsprechende relative Bewegung der distalseitigen Schwenkglieder und somit ein Verschwenken der Werkzeugspitze verursacht, wobei das Bewegen der räumlich verstellbaren Scheibe manuell über eine Art Joystick erfolgt, der direkt mit der räumlich verstellbaren Scheibe gekoppelt ist.

Die Ausbildung des Antriebs für die Lenkdrähte als räumlich verstellbare Scheibe, an der alle vier Lenkdrähte gelagert sind, hat den Vorteil, dass dies eine räumlich kompakte Bauweise ermöglicht und nur ein Bauteil bewegt werden muss, um alle Lenkdrähte ansprechen zu können.

Nachteilig an dieser bekannten Konstruktion ist jedoch einerseits die Verwendung einer nur geringen Anzahl von Lenkdrähten, nämlich von nur vier Lenkdrähten, und andererseits die ausschließlich manuelle Betätigbarkeit der als Antrieb für die Lenkdrähte dienenden räumlich verstellbaren Scheibe, wodurch eine feinfühlige und reproduzierbare Verstellung der distalseitigen Schwenkglieder kaum möglich ist.

Aus der US 2005/0273084 A1 ist weiterhin ein medizinischen Instrument bekannt, bei dem die distalseitigen Schwenkglieder über acht Lenkdrähte mit einem proximalseitig angeordneten Antrieb so gekoppelt, dass eine manuelle Betätigung des Antriebs eine entsprechende relative Bewegung der distalseitigen Schwenkglieder und somit ein Verschwenken der Werkzeugspitze verursacht.

Die US 2006/0020287 A1 offenbart ein medizinischen Instrument bei dem die distalseitigen Schwenkglieder über vier Lenkdrähte mit einem proximalseitig angeordneten Antrieb so gekoppelt sind, dass eine manuelle Betätigung des Antriebs eine entsprechende relative Bewegung der distalseitigen Schwenkglieder und somit ein Verschwenken der Werkzeugspitze verursacht.

Weitere medizinische Instrumente mit über Lenkdrähte verschwenkbaren Werkzeugspitzen sind aus der US 2016/0066982 A1, der US 2015/0352728 A1 sowie der US 2015/0321343 A1 bekannt.

Davon ausgehend liegt der Erfindung die **Aufgabe** zugrunde, ein medizinisches Instrument der eingangs genannten Art zu schaffen, über das eine beliebige Anzahl von Lenkdrähten exakt und reproduzierbar ansteuerbar ist.

Die **Lösung** dieser Aufgabenstellung ist erfindungsgemäß dadurch gekennzeichnet, dass der Antrieb für die räumlich verstellbare Scheibe als motorisierter Antrieb ausgebildet ist, wobei der motorisierte Antrieb aus zwei um 180° zueinander versetzt angeordneten Antriebseinheiten besteht, deren Antriebswellen auf einer gemeinsamen Mittelachse liegen und wobei die räumlich verstellbare Scheibe zwischen den zwei Antriebseinheiten angeordnet ist.

Durch den erfindungsgemäße motorisierten Antrieb der räumlich verstellbaren Scheibe ist es möglich, die Lenkdrähte zum Verschwenken der distalseitigen Schwenkglieder bzw. der Werkzeugspitze exakt, feinfühlig in kleinsten Schritten und auch reproduzierbar zu aktivieren. Darüber hinaus ist die Anzahl der zu verwendenden Lenkdrähte für einen motorisierten Antrieb unerheblich.

Durch die Verwendung von nur zwei Antrieben lässt sich die räumlich verstellbare Scheibe so bewegen, dass die mit den Lenkdrähten gekoppelte Werkzeugspitze in alle Raumrichtungen relativ zur Längsachse des Schaftes verschwenkbar ist. Vorteilhafterweise sind die beiden Antriebseinheiten als angetriebene Zahnräder, insbesondere Kegelräder, ausgebildet.

Gemäß einer bevorzugten Ausführungsform der Erfindung wird vorgeschlagen, dass die räumlich verstellbare Scheibe mit einem dritten Zahnrad gekoppelt ist, das mit den beiden angetriebenen Zahnrädern in Eingriff steht und dessen Drehachse die Mittelachse der als angetriebene Zahnräder ausgebildeten Antriebseinheiten schneidet. Durch die drei miteinander kämmenden Zahnräder wird jede Bewegung der beiden angetriebenen Zahnräder direkt auf das mit der räumlich verstellbaren Scheibe gekoppelte dritte Zahnrad übertragen was seinerseits eine direkte Betätigung der Lenkdrähte bewirkt.

Um die räumlich verstellbare Scheibe trotz der drehfesten Kopplung mit dem dritten Zahnrad dreidimensional verstellen zu können, wird gemäß einer bevorzugten Ausführungsform der Erfindung vorgeschlagen, dass die räumlich verstellbare Scheibe kardanisch auf einer koaxial zur Längsachse des Schaftes verlaufenden Hauptwelle gelagert ist.

Zur Ausbildung der kardanischen Lagerung der räumlich verstellbaren Scheibe wird erfindungsgemäß vorgeschlagen, dass die räumlich verstellbare Scheibe über zwei um 180° versetzt zueinander angeordnete Lagerstifte verschwenkbar auf einer Kreuzgelenkscheibe gelagert ist, wobei die Kreuzgelenkscheibe über zwei um 180° versetzt zueinander angeordnete Lagerstifte verschwenkbar auf der Hauptwelle gelagert ist und wobei die Lagerstifte der räumlich verstellbaren Scheibe und der Kreuzgelenkscheibe um 90° versetzt zueinander angeordnet sind. Diese Lagerung ermöglicht es, die räumlich verstellbare Scheibe um zwei rechtwinklig zueinander stehende Achsen relativ zur Längsachse des Schaftes zu verschwenken, wodurch über die Lenkdrähte distalseitig die Werkzeugspitze in alle Raumrichtungen relativ zur Längsachse des Schaftes verschwenkbar ist.

Um weiterhin zu ermöglichen, dass die Werkzeugspitze zusätzlich zum Verschwenken relativ zur Längsachse des Schaftes auch um die Längsachse des Schaftes rotierbar ist, wird mit der Erfindung vorgeschlagen, dass die räumlich verstellbare Scheibe um die Längsachse des Schaftes rotierbar am dritten Zahnrad gelagert ist, wodurch ein Verdrillen der Lenkdrähte verhindert wird.

Gemäß einer praktischen Ausführungsform der Erfindung wird vorgeschlagen, dass die räumlich verstellbare Scheibe in einem drehfest mit dem dritten Zahnrad gekoppelten Lenkring angeordnet ist.

Weiterhin wird mit der Erfindung vorgeschlagen, dass die räumlich verstellbare Scheibe um die Längsachse des Schaftes rotierbar in einem Lagerring gelagert ist, wobei der Lagerring im Lenkring gelagert ist.

Weiterhin wird mit der Erfindung vorgeschlagen, dass auf der Drehachse des dritten Zahnrads um 180° versetzt zum dritten Zahnrad ein viertes Zahnrad angeordnet ist, das mit den beiden angetriebenen Zahnrädern in Eingriff steht. Dieses vierte Zahnrad schließt die umlaufende Verzahnungskette und sorgt so für eine gleichmäßig umlaufende und spielfreie Kraftverteilung.

Die räumlich verstellbare Scheibe ist erfindungsgemäß über einen Lagerring frei drehbar gegenüber dem vierten Zahnrad mit dem dritten Zahnrad gekoppelt , so dass eine Rotation des vierten Zahnrads um seine Drehachse keine Verdrehung der räumlich verstellbaren Scheibe bewirkt.

Das Festlegen der Lenkdrähte an der räumlich verstellbaren Scheibe erfolgt erfindungsgemäß mittels einer Klemmverbindung, beispielsweise mittels Madenschrauben, die die Lenkdrähte in in der räumlich verstellbaren Scheibe ausgebildeten Durchgangsbohrungen klemmend fixieren.

Gemäß einer weiteren Ausführungsform der Erfindung wird vorgeschlagen, dass distalseitig vor der räumlich verstellbaren Scheibe eine Fächerscheibe auf der Hauptwelle angeordnet ist, die den radialen Abstand der Lenkdrähte von der Längsachse des Schaftes vergrößert. Durch die Vergrößerung des radialen Abstandes der Lenkdrähte von der Längsachse des Schaftes, von beispielsweise einem Durchmesser von 4 mm auf einen Durchmesser von 18 mm, werden nicht nur die Montage und Fertigung des mit der räumlich verstellbaren Scheibe ausgestatten Antriebs der Lenkdrähte vereinfacht, sondern auch die Verstellwinkel der räumlich verstellbaren Scheibe verringert, um eine dem Maß der Durchmesservergrößerung entsprechenden Verschwenkwinkel der Werkzeugspitze zu erzielen.

Mit einer ersten Ausführungsform zur Anordnung der Antriebseinheiten zur Betätigung der räumlich verstellbaren Scheibe wird erfindungsgemäß vorgeschlagen, dass die Mittelachse der beiden als angetriebene Zahnräder ausgebildeten Antriebseinheiten senkrecht zur Längsachse des Schaftes angeordnet ist. Diese Anordnung der Antriebseinheiten, bei der die Längsachse des Schaftes zwischen den beiden Antriebseinheiten hindurch führt, ist konstruktiv besonders einfach herzustellen.

Mit einer alternativen Ausführungsform zur Anordnung der Antriebseinheiten wird mit der Erfindung vorgeschlagen, dass die Mittelachse der beiden als angetriebene Zahnräder ausgebildeten Antriebseinheiten mit der Längsachse des Schaftes zusammenfällt. Diese Ausführungsform zeichnet sich durch eine besonders platzsparende schlanke Bauweise aus.

Schließlich wird mit der Erfindung vorgeschlagen, dass bei der Anordnung der Antriebseinheiten in Längsrichtung der Längsachse des Schaftes die beiden angetriebenen Zahnräder als Hohlzahnräder ausgebildet sind, um einerseits das Betätigungselement zum Betätigen des Werkzeugs in Längsrichtung des Schaftes anordnen zu können und andererseits die Lenkdrähte der räumlich verstellbaren Scheibe zuführen zu können.

Um beim Verschwenken des dritten und vierten Zahnrads relativ zur Längsachse des Schaftes eine Kollision der Zahnräder mit den Lenkdrähten und gegebenenfalls dem Betätigungselement zu vermeiden, wird mit der Erfindung weiterhin vorgeschlagen, dass in den Zahnkränzen des dritten Zahnrads und des vierten Zahnrads Aussparungen für die Lenkdrähte und das Betätigungselement ausgebildet sind.

Weitere Merkmale und Vorteile der Erfindung ergeben sich anhand der zugehörigen Zeichnungen, in denen zwei Ausführungsbeispiele eines erfindungsgemäßen medizinischen Instruments nur beispielhaft dargestellt sind, ohne die Erfindung auf diese Ausführungsbeispiele zu beschränken. In den Zeichnungen zeigt:
- Fig. 1: eine schematische perspektivische Seitenansicht eines erfindungsgemäßen medizinischen Instruments;
- Fig. 2: eine vergrößerte schematische Darstellung einer ersten Ausführungsform des Antriebs gemäß Fig. 1;
- Fig. 3: eine vergrößerte schematische Darstellung einer zweiten Ausführungsform des Antriebs gemäß Fig. 1;
- Fig. 4a: eine vergrößerte perspektivische Detailansicht eines Antriebs gemäß Fig. 2, den Antrieb in einer neutralen Ausgangsstellung darstellend;
- Fig. 4b: eine teilweise geschnittene Darstellung der Ansicht gemäß Fig. 4a ;
- Fig. 5: einen Ansicht gemäß Fig. 4, jedoch den Antrieb in einer ersten Arbeitsstellung darstellend und
- Fig. 6: einen Ansicht gemäß Fig. 4, jedoch den Antrieb in einer zweiten Arbeitsstellung darstellend.

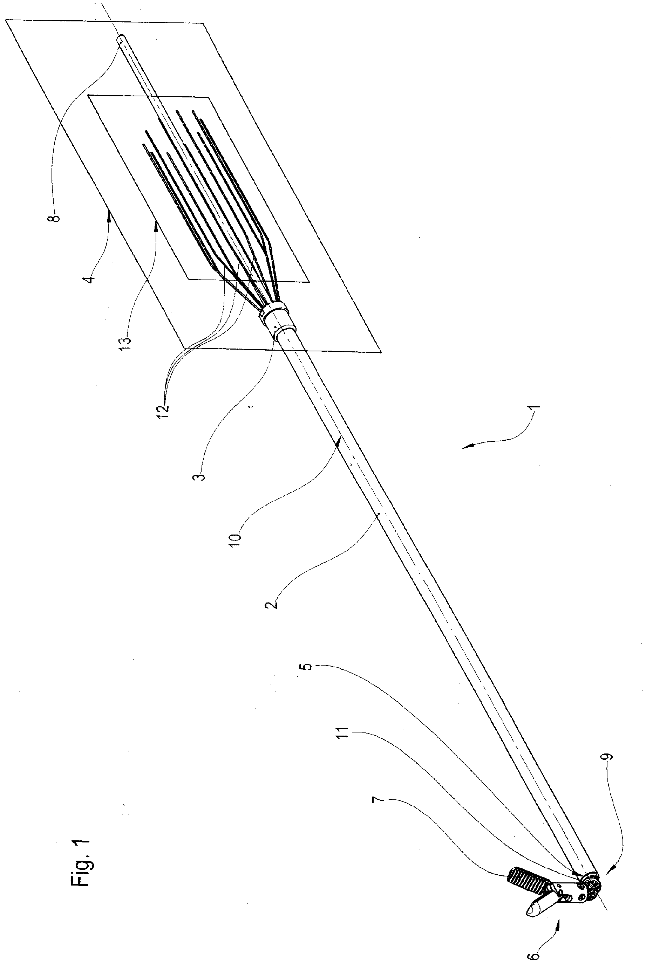

Die Abbildung Fig. 1 zeigt schematisch ein medizinisches Instrument 1 mit einem hohlen Schaft 2, einer am proximalen Ende 3 des Schaftes 2 angeordneten, nur schematisch dargestellten Betätigungseinheit 4 und einer am distalen Ende 5 des Schaftes 2 angeordneten Instrumentenspitze 6 mit einem Instrument 7, wobei das Instrument 7 über ein axial verschiebbar im Schaft 2 gelagertes Betätigungselement 8 betätigbar ist, das proximalseitig mit der Betätigungseinheit 4 in Wirkverbindung steht.

Bei der Betätigungseinheit 4 kann es sich um eine manuell betätigbare Handhabe oder aber um eine für den robotischen Einsatz ausgelegte, also auch ohne manuelles Zutun betätigbare Baueinheit handeln.

Bei dem Instrument 7 der Instrumentenspitze 6 kann es sich, beispielsweise um ein mit Maulteilen versehenes Werkzeug, wie in Fig. 1 dargestellt, oder aber um ein Endoskop, einen Applikator oder dergleichen handeln.

Die Instrumentenspitze 6 ist über einen Gelenkmechanismus 9 relativ zur Längsachse 10 des Schaftes 2 verschwenkbar, wobei der Gelenkmechanismus 9 aus am distalen Ende des Schaftes 5 angeordneten Schwenkgliedern 11 besteht, die über in Längsrichtung des Schaftes 2 verlaufende Lenkdrähte 12 so mit einem am proximalen Ende 3 des Schaftes 2 angeordneten Antrieb 13 verbunden sind, dass eine Bewegung des proximalseitigen Antriebs 13 eine entsprechende relative Bewegung der distalseitigen Schwenkglieder 11 und somit ein Verschwenken der Instrumentenspitze 6 verursacht.

Auch wenn voranstehend und nachfolgend nur der Begriff Lenkdrähte 14 verwendet wird, können funktional auch Lenkseile verwendet werden, weshalb der verwendete Begriff Lenkdrähte 14 synonym auch als Lenkseil zu lesen und zu verstehen ist.

Das axialverschiebbar im Schaft 2 gelagerte Betätigungselement 8 zum Betätigen des beispielsweise aus zwei Maulteilen bestehenden Instruments 7, ist bei den dargestellten Ausführungsformen als Zug-/Schubstange ausgebildet.

Der Antrieb 13 für die Lenkdrähte 12 ist bei dem in den Abbildungen dargestellten und nachfolgend beschriebenen medizinischen Instrument 1 als motorisierter Antrieb 13 ausgebildet.

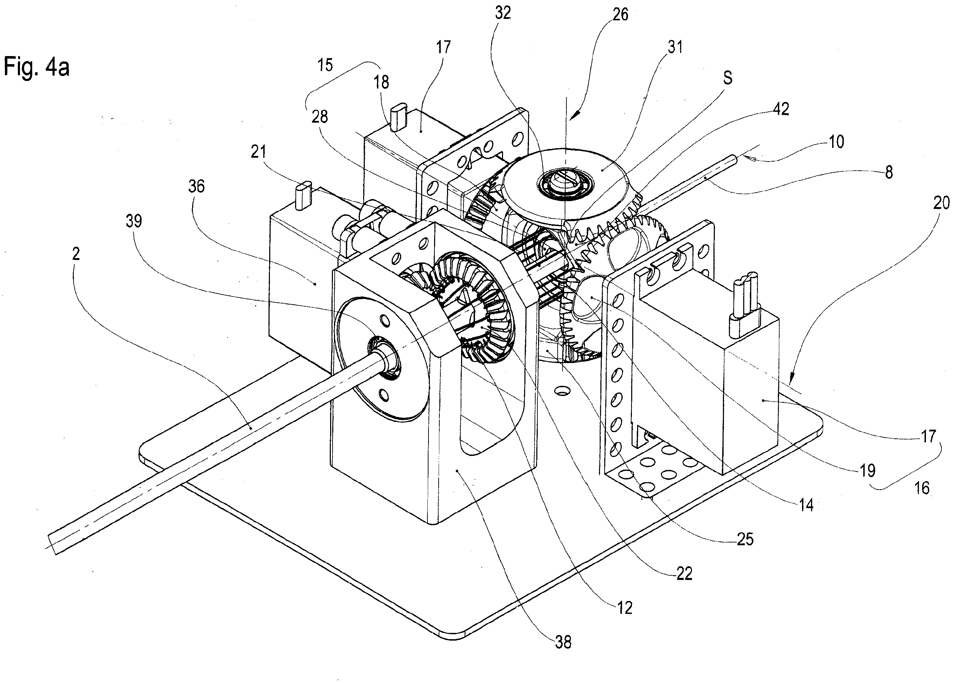

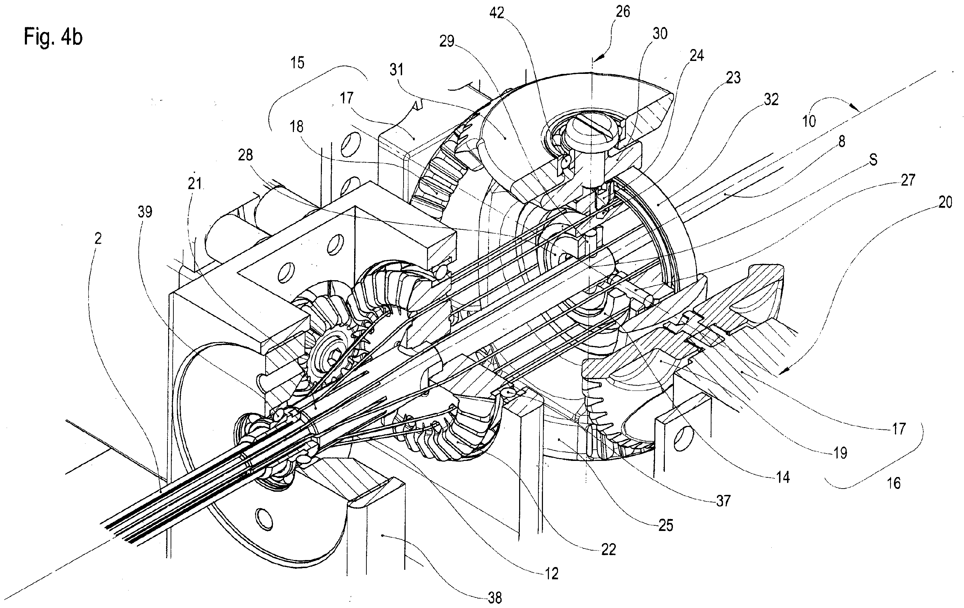

Kernstück des Antriebs 13 ist eine räumlich verstellbare Scheibe 14 (Fig. 4-6), an der die Lenkdrähte 12 so gelagert sind, dass eine Verlagerung der räumlich verstellbaren Scheibe 14 über die an der räumlich verstellbaren Scheibe 14 gelagerten Lenkdrähte 12 ein Verschwenken der Werkzeugspitze 6 bewirkt. Diese räumlich verstellbare Scheibe 14 ist über den motorisierten Antrieb 13 verlagerbar.

Durch die Verwendung eines motorisierten Antriebs 13 für die räumlich verstellbare Scheibe 14 ist es möglich, die Lenkdrähte 12 zum Verschwenken der distalseitigen Schwenkglieder 11 bzw. der Instrumentenspitze 6 exakt, feinfühlig in kleinsten Schritten und auch reproduzierbar anzusteuern . Darüber hinaus ist die Anzahl der zu verwendenden Lenkdrähte 12 für einen motorisierten Antrieb 13 unerheblich.

Der motorisierte Antrieb 13 besteht seinerseits aus zwei um 180° zueinander versetzt angeordneten Antriebseinheiten 15 und 16, deren Antriebswellen auf einer gemeinsamen Mittelachse 20 liegen und wobei die räumlich verstellbare Scheibe 14 zwischen den zwei Antriebseinheiten 15 und 16 angeordnet ist. Die Antriebseinheiten 15 und 16 selber sind als über Motoren 17 angetriebene Zahnräder 18 und 19, vorzugsweise Kegelräder, ausgebildet.

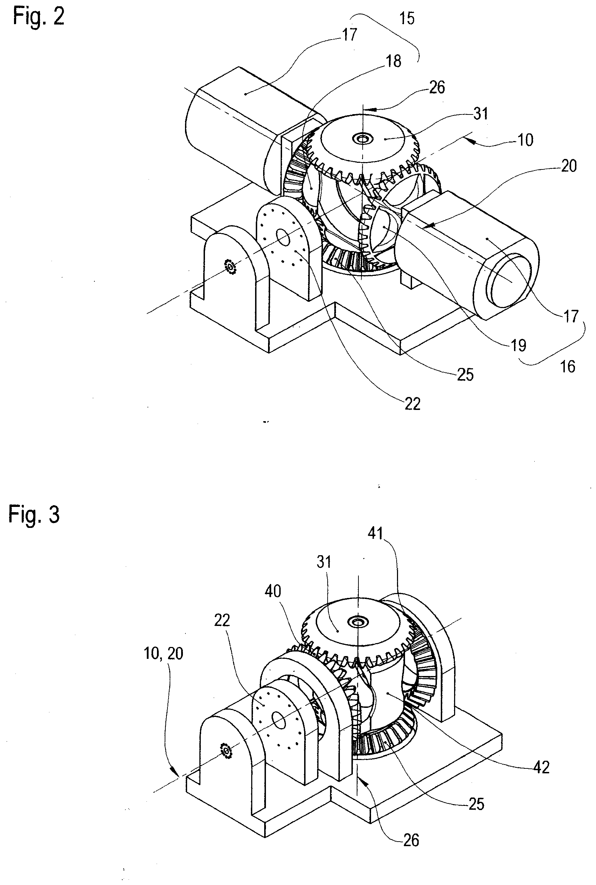

Bei der in den Abbildungen Fig. 2 und 4 bis 6 dargestellten ersten Ausführungsformen des motorisierten Antriebs 13 ist die Mittelachse 20 der beiden Antriebseinheiten 15 und 16 senkrecht zur Längsachse 10 des Schaftes 2 angeordnet ist und kreuzt die Längsachse 10 des Schaftes 2.

Gemäß der in Fig. 3 dargestellten alternativen zweiten Ausführungsform fällt die Mittelachse 20 der beiden Antriebseinheiten 15 und 16 mit der Längsachse 10 des Schaftes 2 zusammen.

Der nachfolgend beschriebene Aufbau der räumlich verstellbaren Scheibe 14 und deren Lagerung sind bei beiden Ausführungsformen des motorisierten Antriebs 13 identisch.

Der Aufbau und der Betrieb des motorisierten Antriebs 13 sowie insbesondere der über die Antriebseinheiten 15 und 16 betätigbaren räumlich verstellbaren Scheibe 14 wird nachfolgend anhand der Abbildungen Fig. 4, 5 und 6 beschrieben.

Wie aus den Abbildungen ersichtlich, ist im Schaft 2 eine sich koaxial zur Längsachse 10 des Schaftes 2 erstreckende hohle Hauptwelle 21 angeordnet, die um die Längsachse 10 des Schaftes 2 rotierbar ist und sich über das proximale Ende 3 des Schaftes 2 hinaus bis in den Bereich des motorisierten Antriebs 13 erstreckt.

Innerhalb dieser hohlen Hauptwelle 21 ist axialverschiebbar das Betätigungselement 8 zur Betätigung des Instruments 7 gelagert.

Die am proximalen Ende 3 des Schaftes 2 aus dem Schaft 2 austretenden Lenkdrähte 12 werden über eine drehfest auf der Hauptwelle 21 angeordnete Fächerscheibe 22 aufgefächert, wodurch der radiale Abstand der Lenkdrähte 12 von der Längsachse 10 des Schaftes 2 vergrößert wird. Während der Durchmesser des die Längsachse 10 des Schaftes 2 koaxial umgebenden Bündels der Lenkdrähte 12 innerhalb des Schaftes 2 beispielsweise 4 mm beträgt, beträgt der Durchmesser des von den Lenkdrähten gebildeten Bündels hinter der Fächerscheibe 22 beispielsweise 18 mm. Bei dem dargestellten Ausführungsbeispiel besteht das Bündel aus zehn einzelnen Lenkdrähten 12.

Proximalseitig hinter der Fächerscheibe 22 werden die parallel zur Längsachse 10 des Schaftes 2 verlaufenden Lenkdrähte 12 der räumlich verstellbaren Scheibe 14 zugeführt. Zum Festlegen der Lenkdrähte 12 an der räumlich verstellbaren Scheibe 14 sind in der räumlich verstellbaren Scheibe 14 Durchgangsbohrungen 23 für jeden Lenkdraht 12 ausgebildet, wobei die Lenkdrähte 12 innerhalb der Durchgangsbohrungen 23 über Madenschrauben 24 fixierbar und kraftschlüssig mit der räumlich verstellbaren Scheibe 14 verbindbar sind.

Die angetriebenen Zahnrädern 18 und 19 sind ihrerseits mit einem dritten Zahnrad 25 gekoppelt, das mit den beiden angetriebenen Zahnrädern 18 und 19 in Eingriff steht und dessen Drehachse 26 die Mittelachse 20 der als angetriebene Zahnräder 18 und 19 ausgebildeten Antriebseinheiten 15 und 16 sowie die Längsachse 10 des Schaftes 2 im Schnittpunkt S schneidet. Auch das dritte Zahnrad 25 ist vorzugsweise als Kegelrad ausgebildet.

Durch die drei miteinander kämmenden Zahnräder 18, 19 und 25 wird jede Bewegung der beiden angetriebenen Zahnräder 18 und 19 direkt auf die mit dem dritten Zahnrad 25 gekoppelte räumlich verstellbare Scheibe 14 übertragen, was seinerseits eine direkte Betätigung der Lenkdrähte 12 bewirkt.

Zur Ausbildung einer kardanischen Lagerung der räumlich verstellbaren Scheibe 14 auf der Hauptwelle 21 ist die räumlich verstellbare Scheibe 14 über zwei um 180° versetzt zueinander angeordnete Lagerstifte 27 verschwenkbar auf einer Kreuzgelenkscheibe 28 gelagert ist, wobei die Kreuzgelenkscheibe 28 über zwei um 180° versetzt zueinander angeordnete Lagerstifte 29 verschwenkbar auf der Hauptwelle 21 gelagert ist. Aus Gründen einer besseren Übersichtlichkeit, ist in den Abbildungen Fig. 4-6 jeweils nur ein Lagerstift 27 und ein Lagerstift 29 dargestellt.

Die Lagerstifte 27 der räumlich verstellbaren Scheibe 14 und die Lagerstifte 29 der Kreuzgelenkscheibe 28 sind dabei um 90° versetzt zueinander angeordnet. Diese Lagerung ermöglicht es, die räumlich verstellbare Scheibe 14 um zwei rechtwinklig zueinander stehende Achsen relativ zur Längsachse 10 des Schaftes 2 zu verschwenken, wodurch über die Lenkdrähte 12 distalseitig die Werkzeugspitze 6 in alle Raumrichtungen relativ zur Längsachse 10 des Schaftes 2 verschwenkbar ist.

Wie weiterhin aus den Abbildungen Fig. 4 -6 ersichtlich, ist die räumlich verstellbare Scheibe 14 in einem drehfest mit dem dritten Zahnrad 25 gekoppelten Lenkring 30 gelagert.

Um die durch die Zahnräder 18, 19 und 25 gebildete Verzahnungskette zu einem geschlossenen Verzahnungsring zu schließen, der eine gleichmäßig umlaufende Kraftverteilung gewährleistet, ist auf der Drehachse 26 des dritten Zahnrads 25 um 180° versetzt zum dritten Zahnrad 25 ein viertes Zahnrad 31 angeordnet, das mit den beiden angetriebenen Zahnrädern 18 und 19 in Eingriff steht, wobei auch das vierte Zahnrad 31 vorzugsweise als Kegelrad ausgebildet ist.

Die räumlich verstellbare Scheibe 14 ist über einen Lagerring 32 in dem drehfest mit dem dritten Zahnrad 25 gekoppelten Lenkring 30 gelagert, um eine Rotation der räumlich verstellbaren Scheibe 14 um die Längsachse 10 des Schaftes 2 zu ermöglichen.

Der drehfest mit dem dritten Zahnrad 25 gekoppelte Lenkring 30 ist über einen Lagerring 42 frei drehbar gegenüber dem vierten Zahnrad 31, so dass eine Rotation des vierten Zahnrads 31 um seine Drehachse 26 keine Verdrehung des Lenkrings 30 und der räumlich verstellbaren Scheibe 14 bewirkt.

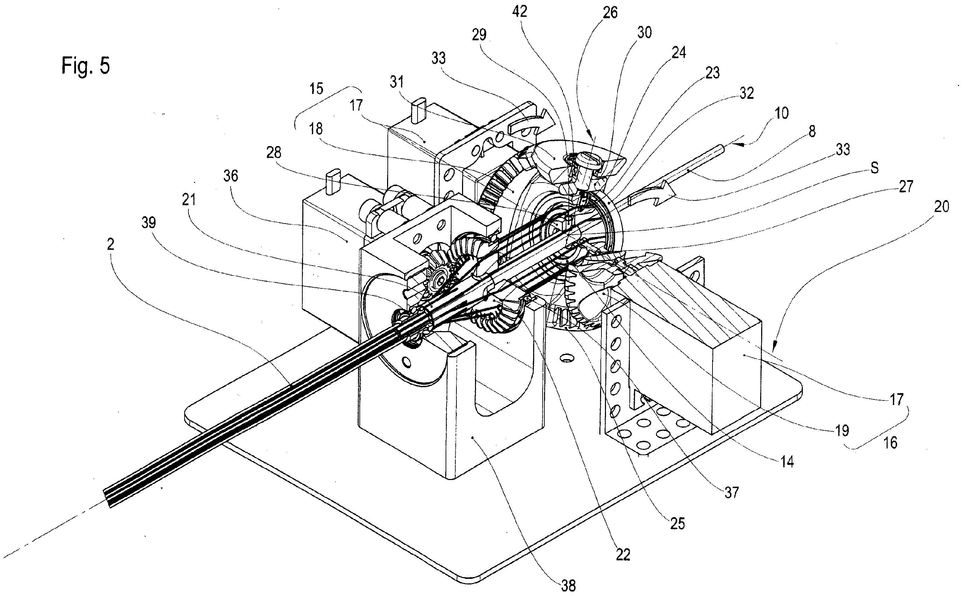

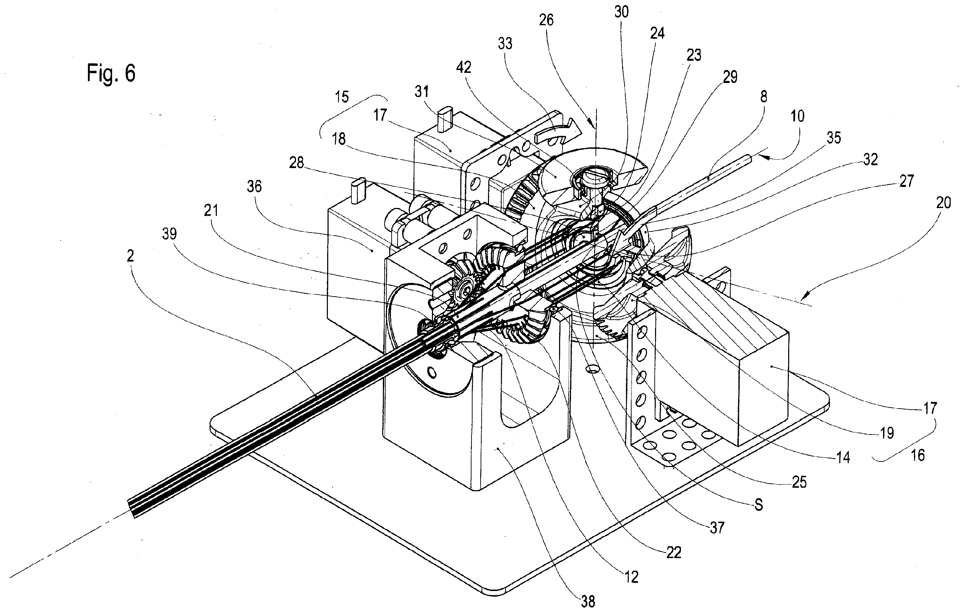

Die beschriebene kardanische Lagerung der räumlich verstellbaren Scheibe 14 auf der Hauptwelle 21 ermöglicht es, die räumlich verstellbare Scheibe 14 dreidimensional relativ zur Längsachse 10 des Schaftes 2 zu verlagern, wie dies in den Abbildungen Fig. 5 und 6 dargestellt ist.

Wenn ausgehend von der in Fig. 4 dargestellten neutralen Ausgangsstellung, in der die räumlich verstellbare Scheibe 14 senkrecht zur Längsachse 10 des Schaftes 2 ausgerichtet ist, die angeriebenen Zahnräder 18 und 19 über die Motoren 17 so angetrieben werden, dass sich die Zahnräder 18 und 19 in dieselbe Richtung drehen, beispielsweise in Richtung der Pfeile 33 in Fig. 5 nach rechts (oder entgegen der Richtung der Pfeile 33 nach links), bewirkt diese Verdrehung der angetriebenen Zahnräder 18 und 19 aufgrund des kämmenden Eingriffs mit dem dritten Zahnrad 25 und dem vierten Zahnrad 31 ein Verkippen der aus dem dritten Zahnrad 25, der mit dem dritten Zahnrad 25 gekoppelten räumlich verstellbaren Scheibe 14 und dem vierten Zahnrad 31 gebildeten Baueinheit um die Mittelachse 20 der angetriebenen Zahnräder 18 und 19, wie dies in Fig. 5 dargestellt ist.

Die mit der Mittelachse 20 der angetriebenen Zahnräder 18 und 19 fluchtenden Lagerstifte 27, über die die räumlich verstellbare Scheibe 14 verschwenkbar an der Kreuzgelenkscheibe 28 gelagert ist, ermöglichen dieses Verkippen der räumlich verstellbaren Scheibe 14 relativ zur Kreuzgelenkscheibe 28.

Dieses Verkippen der räumlich verstellbaren Scheibe 14 um die Mittelachse 20 relativ zur Längsachse 10 des Schaftes 2 bewirkt über die Lenkdrähte 12 das distalseitig die Instrumentenspitze 6 in gleicher Weise relativ zur Längsachse 10 des Schaftes 2 verschwenkt wird.

Wenn ausgehend von der in Fig. 4 dargestellten neutralen Ausgangsstellung, in der die räumlich verstellbare Scheibe 14 senkrecht zur Längsachse 10 des Schaftes 2 ausgerichtet ist, die angeriebenen Zahnräder 18 und 19 über die Motoren 17 so angetrieben werden, dass sich die Zahnräder 18 und 19 in entgegengesetzte Richtungen drehen, beispielsweise das Zahnrad 18 in Richtung des Pfeils 34 in Fig. 6 nach rechts und das Zahnrad 19 in Richtung des Pfeils 35 in Fig. 6 nach links (oder auch umgekehrt), bewirkt diese Verdrehung der angetriebenen Zahnräder 18 und 19 aufgrund des kämmenden Eingriffs mit dem dritten Zahnrad 25 ein Verdrehen der aus dem dritten Zahnrad 25, und der mit dem dritten Zahnrad 25 gekoppelten räumlich verstellbaren Scheibe 14 gebildeten Baueinheit um die Drehachse 26 des dritten Zahnrads 25, wie dies in Fig. 6 dargestellt ist.

Die mit der Drehachse 26 des dritten Zahnrads 25 fluchtenden Lagerstifte 29, über die die Kreuzgelenkscheibe 28 verschwenkbar an der Hauptwelle 21 gelagert ist, ermöglichen zusammen mit der freien Drehbarkeit der räumlich verstellbaren Scheibe 14 relativ zum vierten Zahnrad 31 aufgrund des Lagerrings 32 dieses Verdrehen der Kreuzgelenkscheibe 28 relativ zur Hauptwelle 21.

Dieses Verdrehen der räumlich verstellbaren Scheibe 14 um die Drehachse 26 relativ zur Längsachse 10 des Schaftes 2 bewirkt über die Lenkdrähte 12 das distalseitig die Instrumentenspitze 6 in gleicher Weise relativ zur Längsachse 10 des Schaftes 2 verschwenkt wird.

Zusätzlich zu den in den Abbildungen Fig. 5 und 6 dargestellten Verlagerungen der räumlich verstellbaren Scheibe 14 ist es selbstverständlich auch möglich, die dargestellten Bewegungen zu überlagern, so dass beispielsweise die räumlich verstellbare Scheibe um die Mittlachse 20 verkippt und gleichzeitig auch noch um die Drehachse 26 verdreht wird. Durch diese Kombination der beiden Bewegungsabläufe aufgrund der einzeln ansteuerbaren Motoren 17 des Antriebs 13 lässt sich die räumlich verstellbare Scheibe 14 dreidimensional relativ zur Längsachse 10 des Schaftes 2 verstellen, woraus aufgrund der Kopplung über die Lenkdrähte 12 eine räumliche Verlagerung der Instrumentenspitze 6 resultiert.

Durch die mit Hilfe der Fächerscheibe 22 erzielte Vergrößerung des radialen Abstandes der Lenkdrähte 12 von der Längsachse 10 des Schaftes 2, werden nicht nur die Montage und Fertigung des mit der räumlich verstellbaren Scheibe 14 ausgestatten Antriebs 13 der Lenkdrähte 14 vereinfacht, sondern wird auch notwendige der Verstellwinkel der räumlich verstellbaren Scheibe 14 proportional verringert, um einen erwünscht hohen Verschwenkwinkel der Instrumentenspitze 6 zu erzielen.

Wenn beispielsweise der Durchmesser des von den Lenkdrähten 12 gebildeten Bündels von 4 mm innerhalb des Schaftes 2 auf 18 mm hinter der Fächerscheibe 22 vergrößert wird, also um das 4,5-fache, verringert sich der Verstellwinkel der räumlich verstellbaren Scheibe 14 um das 4,5-fache, gegenüber dem am distalen Ende erzielbaren Verschwenkwinkel der Instrumentenspitze 6. Um die Werkzeugspitze 6 um 90°abzuwinkeln, bedarf es somit nur einer Verschwenkung der räumlich verstellbaren Scheibe 14 um 20°.

Um die Hauptwelle 12 in eine Rotation um die Längsachse 10 des Schaftes 2 zu versetzen, in deren Folge auch die distalseitig mit der Hauptwelle 21 gekoppelte Instrumentenspitze 6 um die Längsachse 10 des Schaftes 2 rotiert, ist bei der in Fig. 4-6 dargestellten Ausführungsform des medizinischen Instruments 1 die drehfest auf der Hauptwelle 21 angeordnete Fächerscheibe 22 als über einen Motor 36 antreibbares Zahnrad ausgebildet, das über einen Lagerring 37 drehbar in einem Lagerblock 38 der Betätigungseinheit 4 gelagert ist. Ein distalseitig vor der Fächerscheibe 22 angeordneter weiterer Lagerring 39 sorgt für eine stabile Führung der Hauptwelle 21.

Eine Rotation der Hauptwelle 21 um die Längsachse 10 des Schaftes 2 wird über die drehfest auf der Hauptwelle 21 gelagerte Kreuzgelenkscheibe 28 und die Lagerstifte 27 auf die räumlich verstellbare Scheibe 14 übertragen. Für den Fall, dass die räumlich verstellbare Scheibe 14 während der Rotation der Hauptwelle 21 um die Längsachse 10 des Schaftes 2 relativ zur Längsachse 10 des Schaftes 2 verkippt und/oder verdreht ist, durchlaufen die an der räumlich verstellbaren Scheibe 14 festgelegten Lenkdrähte 12 zwar verschiedene Steuerstellungen, die distalseitige Abwinklung der Instrumentenspitze 6 bleibt aber trotz der Rotation der Instrumentenspitze 6 um die Längsachse 10 des Schaftes 2 räumlich gleich.

Bei der in Fig. 3 dargestellten alternativen Anordnung der Antriebseinheiten 15 und 16 in Längsrichtung der Längsachse 10 des Schaftes 2 sind die beiden angetriebenen Zahnräder als Hohlzahnräder 40 und 41 ausgebildet, um einerseits das Betätigungselement 8 zum Betätigen des Instruments 7 in Längsrichtung 10 des Schaftes 2 anordnen zu können und andererseits die Lenkdrähte 12 der räumlich verstellbaren Scheibe 14 zuführen zu können. Bei dieser Konstruktion werden die Hohlzahnräder 40 und 41 über nicht dargestellte seitliche Zahnräder angetrieben.

Ein wie zuvor beschrieben ausgebildetes medizinisches Instrument 1 zeichnet sich dadurch aus, dass viele dünne Lenkdrähte 12 zur Ansteuerung der verschwenkbaren Instrumentenspitze 6 verwendet werden können und diese Ansteuerung aufgrund des motorisierten Antriebs 13 für die räumlich verstellbare Scheibe 14, an der die Lenkdrähte 12 proximalseitig gelagert sind, feinfühlig, exakt und reproduzierbar erfolgt.

### Bezugszeichenliste

- 1: medizinisches Instrument
- 2: Schaft
- 3: proximales Ende (Schaft)
- 4: Betätigungseinheit
- 5: distales Ende (Schaft)
- 6: Instrumentenspitze
- 7: Instrument
- 8: Betätigungselement
- 9: Gelenkmechanismus
- 10: Längsachse
- 11: Schwenkglied
- 12: Lenkdraht
- 13: Antrieb
- 14: Scheibe (räumlich verstellbar)
- 15: Antriebseinheit
- 16: Antriebseinheit
- 17: Motor
- 18: Zahnrad (angetrieben)
- 19: Zahnrad (angetrieben)
- 20: Mittelachse
- 21: Hauptwelle
- 22: Fächerscheibe
- 23: Durchgangsbohrung
- 24: Madenschraube
- 25: drittes Zahnrad
- 26: Drehachse
- 27: Lagerstift
- 28: Kreuzgelenkscheibe
- 29: Lagerstift
- 30: Lenkring
- 31: viertes Zahnrad
- 32: Lagerring
- 33: Pfeil
- 34: Pfeil
- 35: Pfeil
- 36: Motor
- 37: Lagerring
- 38: Lagerblock
- 39: Lagerring
- 40: Hohlzahnrad
- 41: Hohlzahnrad
- 42: Lagerring

- S: Schnittpunkt

## Patentansprüche

1. Medizinisches Instrument mit einem hohlen Schaft (2), einer am proximalen Ende (3) des Schaftes (2) angeordneten Betätigungseinheit (4) und einer am distalen Ende (5) des Schaftes (2) angeordneten Instrumentenspitze (6) mit einem Instrument (7), wobei das Instrument (7) über ein axial verschiebbar im Schaft (2) gelagertes Betätigungselement (8) betätigbar ist, das proximalseitig mit der Betätigungseinheit (4) in Wirkverbindung steht und die Instrumentenspitze (6) über einen Gelenkmechanismus (9) relativ zur Längsachse (10) des Schaftes (2) verschwenkbar ist, wobei der Gelenkmechanismus (9) aus am distalen Ende (5) des Schaftes (2) angeordneten Schwenkgliedern (11) besteht, die über in Längsrichtung des Schaftes (2) verlaufende Lenkdrähte (12) so mit einem proximalseitigen Antrieb (13) verbunden sind, dass eine Bewegung des proximalseitigen Antriebs (13) eine entsprechende relative Bewegung der distalseitigen Schwenkglieder (11) und somit ein Verschwenken der Instrumentenspitze (6) verursacht und wobei der proximalseitige Antrieb (13) eine räumlich verstellbare Scheibe (14) aufweist, an der die Lenkdrähte (12) gelagert sind,
**dadurch gekennzeichnet,**
**dass** der Antrieb (13) für die räumlich verstellbare Scheibe (14) als motorisierter Antrieb (13) ausgebildet ist, wobei der motorisierte Antrieb (13) aus zwei um 180° zueinander versetzt angeordneten Antriebseinheiten (15, 16) besteht, deren Antriebswellen auf einer gemeinsamen Mittelachse (20) liegen und wobei die räumlich verstellbare Scheibe (14) zwischen den zwei Antriebseinheiten (15, 16) angeordnet ist.

2. Medizinisches Instrument nach Anspruch 1, **dadurch gekennzeichnet, dass** die Antriebseinheiten (15, 16) als angetriebene Zahnräder (18, 19) ausgebildet sind.

3. Medizinisches Instrument nach Anspruch 1, **dadurch gekennzeichnet, dass** die räumlich verstellbare Scheibe (14) mit einem dritten Zahnrad (25) gekoppelt ist, das mit den beiden angetriebenen Zahnrädern (18, 19) in Eingriff steht und dessen Drehachse (26) die Mittelachse (20) der als angetriebene Zahnräder (18, 19) ausgebildeten Antriebseinheiten (15, 16) schneidet.

4. Medizinisches Instrument nach Anspruch 2 oder 3, **dadurch gekennzeichnet, dass** die räumlich verstellbare Scheibe (14) kardanisch auf einer koaxial zur Längsachse (10) des Schaftes (2) verlaufenden Hauptwelle (21) gelagert ist.

5. Medizinisches Instrument nach Anspruch 4, **dadurch gekennzeichnet, dass** die räumlich verstellbare Scheibe (14) über zwei um 180° versetzt zueinander angeordnete Lagerstifte (27) verschwenkbar auf einer Kreuzgelenkscheibe (28) gelagert ist, wobei die Kreuzgelenkscheibe (28) über zwei um 180° versetzt zueinander angeordnete Lagerstifte (29) verschwenkbar auf der Hauptwelle (21) gelagert ist und wobei die Lagerstifte (27 und 29) der räumlich verstellbaren Scheibe (14) und der Kreuzgelenkscheibe (28) um 90° versetzt zueinander angeordnet sind.

6. Medizinisches Instrument nach Anspruch 4 oder 5, **dadurch gekennzeichnet, dass** die räumlich verstellbare Scheibe (14) um die Längsachse (10) des Schaftes (2) rotierbar am dritten Zahnrad (25) gelagert ist.

7. Medizinisches Instrument nach Anspruch 6, **dadurch gekennzeichnet, dass** die räumlich verstellbare Scheibe (14) in einem drehfest mit dem dritten Zahnrad (25) gekoppelten Lenkring (30) angeordnet ist.

8. Medizinisches Instrument nach Anspruch 7, **dadurch gekennzeichnet, dass** die räumlich verstellbare Scheibe (14) um die Längsachse (10) des Schaftes (2) rotierbar in einem Lagerring (32) gelagert ist, wobei der Lagerring (32) im Lenkring (30) gelagert ist.

9. Medizinisches Instrument nach einem der Ansprüche 3 bis 8, **dadurch gekennzeichnet, dass** auf der Drehachse (26) des dritten Zahnrads (25) um 180° versetzt zum dritten Zahnrad (25) ein viertes Zahnrad (31) angeordnet ist, das mit den beiden angetriebenen Zahnrädern (18, 19) in Eingriff steht.

10. Medizinisches Instrument nach Anspruch 9, **dadurch gekennzeichnet, dass** die räumlich verstellbare Scheibe (14) über einen Lagerring (42) frei drehbar gegenüber dem vierten Zahnrad (31) mit dem dritten Zahnrad (25) gekoppelt ist.

11. Medizinisches Instrument nach einem der Ansprüche 5 bis 10, **dadurch gekennzeichnet, dass** distalseitig vor der räumlich verstellbaren Scheibe (14) eine Fächerscheibe (22) auf der Hauptwelle (21) angeordnet ist, die den radialen Abstand der Lenkdrähte (12) von der Längsachse (10) des Schaftes (2) vergrößert.

12. Medizinisches Instrument nach einem der Ansprüche 2 bis 11, **dadurch gekennzeichnet, dass** die Mittelachse (20) der beiden als angetriebene Zahnräder (18, 19) ausgebildeten Antriebseinheiten (15, 16) senkrecht zur Längsachse (10) des Schaftes (2) angeordnet ist.

13. Medizinisches Instrument nach einem der Ansprüche 2 bis 11, **dadurch gekennzeichnet, dass** die Mittelachse (20) der beiden als angetriebene Zahnräder (18, 19) ausgebildeten Antriebseinheiten (15, 16) mit der Längsachse (10) des Schaftes (2) zusammenfällt.

14. Medizinisches Instrument nach Anspruch 13, **dadurch gekennzeichnet, dass** die beiden angetriebenen Zahnräder (18, 19) als Hohlzahnräder (40, 41) ausgebildet sind.

15. Medizinisches Instrument nach einem der Ansprüche 9 bis 14, **dadurch gekennzeichnet, dass** in den Zahnkränzen des dritten Zahnrads (25) und des vierten Zahnrads (31) Aussparungen für die Lenkdrähte (12) und das Betätigungselement (8) ausgebildet sind.

## Claims

1. Medical instrument with a hollow shaft (2), an actuating unit (4) arranged at the proximal end (3) of the shaft (2), and an instrument tip (6) with an instrument (7) arranged at the distal end (5) of the shaft (2), wherein the instrument (7) is actuatable via an actuating element (8), which is mounted axially displaceably in the shaft (2) and is operatively connected at the proximal end to the actuating unit (4), and the instrument tip (6) is pivotable relative to the longitudinal axis (10) of the shaft (2) via a joint mechanism (9), wherein the joint mechanism (9) is composed of pivoting members (11) which are arranged at the distal end (5) of the shaft (2) and which, via steering wires (12) running in the longitudinal direction of the shaft (2), are connected to a proximal-side drive (13) in such a way that a movement of the proximal-side drive (13) causes a corresponding relative movement of the distal-side pivoting members (11) and thus a pivoting of the instrument tip (6), and wherein the proximal-side drive (13) has a spatially adjustable disc (14) on which the steering wires (12) are mounted,
**characterized in that**
the drive (13) for the spatially adjustable disc (14) is designed as a motorized drive (13), wherein the motorized drive (13) is composed of two drive units (15, 16) which are arranged offset from each other by 180° and of which the drive shafts lie on a common central axis (20), and wherein the spatially adjustable disc (14) is arranged between the two drive units (15, 16) .

2. Medical instrument according to Claim 1, **characterized in that** the drive units (15, 16) are designed as driven gearwheels (18, 19).

3. Medical instrument according to Claim 1, **characterized in that** the spatially adjustable disc (14) is coupled to a third gearwheel (25) which engages with the two driven gearwheels (18, 19) and whose axis of rotation (26) intersects the central axis (20) of the drive units (15, 16) designed as driven gearwheels (18, 19).

4. Medical instrument according to Claim 2 or 3, **characterized in that** the spatially adjustable disc (14) is mounted cardanically on a main shaft (21) running coaxially with respect to the longitudinal axis (10) of the shaft (2).

5. Medical instrument according to Claim 4, **characterized in that** the spatially adjustable disc (14) is mounted pivotably on a universal joint disc (28) via two bearing pins (27) which are offset from each other by 180°, wherein the universal joint disc (28) is mounted pivotably on the main shaft (21) via two bearing pins (29) which are offset from each other by 180°, and wherein the bearing pins (27 and 29) of the spatially adjustable disc (14) and of the universal joint disc (28) are arranged offset from each other by 90° .

6. Medical instrument according to Claim 4 or 5, **characterized in that** the spatially adjustable disc (14) is mounted on the third gearwheel (25) rotatably about the longitudinal axis (10) of the shaft (2).

7. Medical instrument according to Claim 6, **characterized in that** the spatially adjustable disc (14) is arranged in a steering ring (30) which is coupled to the third gearwheel (25) in a rotationally fixed manner.

8. Medical instrument according to Claim 7, **characterized in that** the spatially adjustable disc (14) is mounted in a bearing ring (32) rotatably about the longitudinal axis (10) of the shaft (2), wherein the bearing ring (32) is mounted in the steering ring (30) .

9. Medical instrument according to one of Claims 3 to 8, **characterized in that** a fourth gearwheel (31) is arranged on the axis of rotation (26) of the third gearwheel (25) in a manner offset from the third gearwheel (25) by 180°, which fourth gearwheel (31) engages with the two driven gearwheels (18, 19).

10. Medical instrument according to Claim 9, **characterized in that** the spatially adjustable disc (14) is coupled to the third gearwheel (25) via a bearing ring (42) in a freely rotatable manner with respect to the fourth gearwheel (31).

11. Medical instrument according to one of Claims 5 to 10, **characterized in that** a serrated lock washer (22) is arranged on the main shaft (21) on the distal side in front of the spatially adjustable disc (14), which serrated lock washer (22) increases the radial distance of the steering wires (12) from the longitudinal axis (10) of the shaft (2).

12. Medical instrument according to one of Claims 2 to 11, **characterized in that** the central axis (20) of the two drive units (15, 16) designed as driven gearwheels (18, 19) is arranged perpendicular to the longitudinal axis (10) of the shaft (2).

13. Medical instrument according to one of Claims 2 to 11, **characterized in that** the central axis (20) of the two drive units (15, 16) designed as driven gearwheels (18, 19) coincides with the longitudinal axis (10) of the shaft (2).

14. Medical instrument according to Claim 13, **characterized in that** the two driven gearwheels (18, 19) are designed as hollow gearwheels (40, 41).

15. Medical instrument according to one of Claims 9 to 14, **characterized in that** recesses for the steering wires (12) and the actuating element (8) are formed in the toothed rings of the third gearwheel (25) and of the fourth gearwheel (31).

## Revendications

1. Instrument médical comprenant une tige creuse (2), une unité d'actionnement (4) disposée à l'extrémité proximale (3) de la tige (2) et une pointe d'instrument (6) comprenant un instrument (7) disposé à l'extrémité distale (5) de la tige (2), l'instrument (7) pouvant être actionné par le biais d'un élément d'actionnement (8) qui est monté de manière déplaçable axialement dans la tige (2) et qui est en liaison fonctionnelle du côté proximal avec l'unité d'actionnement (4) et la pointe d'instrument (6) pouvant pivoter par rapport à l'axe longitudinal (10) de la tige (2) par le biais d'un mécanisme d'articulation (9), le mécanisme d'articulation (9) comprenant des éléments de pivotement (11) qui sont disposés à l'extrémité distale (5) de la tige (2) et qui sont reliés à un entraînement côté proximal (13) par le biais de fils de direction (12) dans la direction longitudinale de la tige (2) de sorte qu'un mouvement de l'entraînement côté proximal (13) génère un mouvement relatif correspondant des éléments de pivotement côté distal (11) et ainsi un pivotement de la pointe d'instrument (6) et l'entraînement côté proximal (13) comportant un disque (14) réglable dans l'espace sur lequel les fils de direction (12) sont montés,
**caractérisé en ce que**
l'entraînement (13) destiné au disque (14) réglable dans l'espace est conçu comme un entraînement motorisé (13), l'entraînement motorisé (13) comprenant deux unités d'entraînement (15, 16) disposées de manière décalée de 180° l'une de l'autre, dont les arbres d'entraînement sont situés sur un axe central commun (20) et le disque (14) réglable dans l'espace étant disposé entre les deux unités d'entraînement (15, 16).

2. Instrument médical selon la revendication 1, **caractérisé en ce que** les unités d'entraînement (15, 16) sont conçues comme des roues dentées menées (18, 19) .

3. Instrument médical selon la revendication 1, **caractérisé en ce que** le disque (14) réglable spatialement est accouplé à une troisième roue dentée (25) qui s'engrène avec les deux roues dentées menées (18, 19) et dont l'axe de rotation (26) coupe l'axe central (20) des unités d'entraînement (15, 16) conçues comme des roues dentées menées (18, 19).

4. Instrument médical selon la revendication 2 ou 3, **caractérisé en ce que** le disque (14) réglable spatialement est monté par le biais d'un cardan sur un arbre principal (21) s'étendant coaxialement à l'axe longitudinal (10) de la tige (2).

5. Instrument médical selon la revendication 4, **caractérisé en ce que** le disque (14) réglable spatialement est monté de manière pivotante sur un disque à cardan (28) par le biais de deux tourillons (27) disposés de manière décalée de 180° l'un de l'autre, le disque de cardan (28) étant monté de manière pivotante sur l'arbre principal (21) par le biais de deux tourillons (29) disposés de manières décalée de 180° l'un de l'autre, et les tourillons (27 et 29) du disque (14) réglable dans l'espace et du disque à cardan (28) étant disposés de manière décalée de 90° l'un de l'autre.

6. Instrument médical selon la revendication 4 ou 5, **caractérisé en ce que** le disque (14) réglable spatialement est monté sur la troisième roue dentée (25) de manière rotative sur l'axe longitudinal (10) de la tige (2).

7. Instrument médical selon la revendication 6, **caractérisé en ce que** le disque (14) réglable dans l'espace est disposé dans une bague de direction (30) qui est accouplée solidairement en rotation à la troisième roue dentée (25).

8. Instrument médical selon la revendication 7, **caractérisé en ce que** le disque (14) réglable spatialement est monté dans une bague de palier (32) de manière rotative sur l'axe longitudinal (10) de la tige (2), la bague de palier (32) étant montée dans la bague de direction (30).

9. Instrument médical selon l'une des revendications 3 à 8, **caractérisé en ce qu'**une quatrième roue dentée (31), qui s'engrène avec les deux roues dentées menées (18, 19), est disposée sur l'axe de rotation (26) de la troisième roue dentée (25) de manière décalée de 180° par rapport à la troisième roue dentée (25).

10. Instrument médical selon la revendication 9, **caractérisé en ce que** le disque (14) réglable spatialement est accouplé à la troisième roue dentée (25) par le biais d'une bague de palier (42) de manière à pouvoir tourner librement par rapport à la quatrième roue dentée (31).

11. Instrument médical selon l'une des revendications 5 à 10, **caractérisé en ce qu'**une rondelle en éventail (22), qui augmente la distance radiale des fils de direction (12) à l'axe longitudinal (10) de la tige (2), est disposée sur l'arbre principal (21) du côté distal en amont du disque (14) spatialement réglable.

12. Instrument médical selon l'une des revendications 2 à 11, **caractérisé en ce que** l'axe central (20) des deux unités d'entraînement (15, 16) conçues comme des roues dentées menées (18, 19) est disposé perpendiculairement à l'axe longitudinal (10) de la tige (2).

13. Instrument médical selon l'une des revendications 2 à 11, **caractérisé en ce que** l'axe central (20) des deux unités d'entraînement (15, 16) conçues comme des roues dentées menées (18, 19) coïncide avec l'axe longitudinal (10) de la tige (2).

14. Instrument médical selon la revendication 13, **caractérisé en ce que** les deux roues dentées menées (18, 19) sont conçues comme des couronnes à denture intérieure (40, 41).

15. Instrument médical selon l'une des revendications 9 à 14, **caractérisé en ce que** des évidements destinés à des fils de direction (12) et l'élément d'actionnement (8) sont formés dans les couronnes dentées de la troisième roue dentée (25) et de la quatrième roue dentée (31).
